# EUROPEAN PATENT APPLICATION

(11) **EP 0 622 464 A2**
(43) Date of publication of application: **02.11.1994**
(21) Application number: 94105098.1
(22) Date of filing: 31.03.1994
(51) Int. Cl.: C12Q 1/68

(54) **Nucleic acid assay procedure**

(30) Priority: 16.04.1993 US 50683
(71) Applicant: Becton Dickinson and Company, Franklin Lakes, New Jersey 07417-1880 (US)
(72) Inventor: Nycz, Colleen Marie, Raleigh, North Carolina 27613 (US); Vonk, Glenn P., Fuquay-Varina, North Carolina 27526 (US); Jurgensen, Stewart Russell, Raleigh, North Carolina 27615 (US); Myatich, Ronald G,., Durham, North Carolina 27113 (US)
(74) Representative: Rambelli, Paolo

(57) **Abstract**

A method of detecting the presence of a target polynucleotide in a sample is disclosed. The method comprises the steps of hybridizing the target polynucleotide with a detector probe, the detector probe comprising a first oligonucleotide having a detectable group such as alkaline phosphatase attached to the 3' terminus thereof; hybridizing the target polynucleotide with a capture probe, the capture probe comprising a second oligonucleotide having a capture group such as biotin attached to the 5' terminus thereof; and detecting the hybridization of the detector probe and the capture probe to the target polynucleotide. Probes, probe sets, and kits useful for carrying out the invention are also disclosed.

SEQUENCE LISTING

(1) GENERAL INFORMATION:

(i) APPLICANT: BECTON, DICKINSON AND COMPANY

(A) NAME: BECTON, DICKINSON AND COMPANY
(B) STREET: 1 Becton Drive
(C) CITY: Franklin Lakes
(D) STATE: New Jersey
(E) COUNTRY: USA
(F) ZIP: 07417-1880

(ii) TITLE OF INVENTION: Nucleic Acid Assay Procedure

(iii) NUMBER OF SEQUENCES: 14

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(v) CURRENT APPLICATION DATA:
(A) APPLICATION NUMBER:
(B) FILING DATE:

(2) INFORMATION FOR SEQ ID NO:1:

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:1:

GGGAACCGGT GACTC 15

(2) INFORMATION FOR SEQ ID NO:2:

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:2:

CAAAAACCTT GCGGC 15

(2) INFORMATION FOR SEQ ID NO:3:

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:3:

GGGAACCGGT GACTC 15

(2) INFORMATION FOR SEQ ID NO:4:

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:4:

CAAAAACCTT GCGGC 15

(2) INFORMATION FOR SEQ ID NO:5:

(i) SEQUENCE CHARACTERISTICS:
(A) LENGTH: 66 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:5:

GACCCGACTT GTAAGAGCCG CAAGGTTTTT GGAGTCACCG GTTCCCACTC GCAGCCTGCG 60

TCTTTT                                                    66

(2) INFORMATION FOR SEQ ID NO:6:

     (i) SEQUENCE CHARACTERISTICS:
          (A) LENGTH: 14 base pairs
          (B) TYPE: nucleic acid
          (C) STRANDEDNESS: single
          (D) TOPOLOGY: linear

    (ii) MOLECULE TYPE: cDNA

    (xi) SEQUENCE DESCRIPTION: SEQ ID NO:6:

AACCGGTGAC TCCA                                           14

(2) INFORMATION FOR SEQ ID NO:7:

     (i) SEQUENCE CHARACTERISTICS:
          (A) LENGTH: 13 base pairs
          (B) TYPE: nucleic acid
          (C) STRANDEDNESS: single
          (D) TOPOLOGY: linear

    (ii) MOLECULE TYPE: cDNA

    (xi) SEQUENCE DESCRIPTION: SEQ ID NO:7:

AAAACCTTGC GGC                                            13

(2) INFORMATION FOR SEQ ID NO:8:

     (i) SEQUENCE CHARACTERISTICS:
          (A) LENGTH: 13 base pairs
          (B) TYPE: nucleic acid
          (C) STRANDEDNESS: single
          (D) TOPOLOGY: linear

    (ii) MOLECULE TYPE: cDNA

    (xi) SEQUENCE DESCRIPTION: SEQ ID NO:8:

AAAACCTTGC GGC                                            13

(2) INFORMATION FOR SEQ ID NO:9:

     (i) SEQUENCE CHARACTERISTICS:
          (A) LENGTH: 14 base pairs
          (B) TYPE: nucleic acid
          (C) STRANDEDNESS: single
          (D) TOPOLOGY: linear

    (ii) MOLECULE TYPE: cDNA

    (xi) SEQUENCE DESCRIPTION: SEQ ID NO:9:

AACCGGTGAC TCCA 14

(2) INFORMATION FOR SEQ ID NO:10:

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:10:

TATCCACCAT ACGGA 15

(2) INFORMATION FOR SEQ ID NO:11:

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:11:

CGACCTGAAA GACGT 15

(2) INFORMATION FOR SEQ ID NO:12:

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:12:

CCTGAAAGAC GTTAT 15

(2) INFORMATION FOR SEQ ID NO:13:

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:13:

CCACCATACG GATAG 15

(2) INFORMATION FOR SEQ ID NO:14:

(i) SEQUENCE CHARACTERISTICS:
(A) LENGTH: 63 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:14:

GACACTGAGA TCCCCTATCC GTATGGTGGA TAACGTCTTT CAGGTCGAGT ACGCCGTCTT 60

TTT 63

## Description

### Field of the Invention

This invention relates to the detection of genetic material with oligonucleotide probes in sandwich assays.

### Background of the Invention

One known strategy for detecting the presence of a target polynucleotide within a sample is called the sandwich assay. A sandwich assay employs separate detector and capture oligonucleotides, each of which are complimentary to a base pair sequence of the target polynucleotide of interest. In the presence of the target polynucleotide, the detector and capture oligonucleotides each hybridize to a complimentary base pair sequence. Typically, the detector oligonucleotide will be conjugated to a moiety that is capable of producing a detectable signal upon contact with a particular signalling substrate. The capture oligonucleotide is fixed either directly or through a fixing moiety to a solid support. After removing excess sample and other reagents from the solid support, the capture-target-detector complex is exposed to the signalling substrate. The intensity of the signal produced by the signalling substrate indicates the concentration of the target polynucleotide present in the sample.

When a signalling moiety is employed, it is generally attached to either the internal portion of the polynucleotide (as is the case when radioactive labels are added to the detector probe through chemical "nicking" of nucleotides) or to the 5' terminus of the detector. A common problem that has affected the sensitivity of sandwich assays is the presence of non-specific signal in the assay. Non-specific signalling can be due to retention of unhybridized detector probe in the solution, etc. Non-specific signal can skew the results of an assay by increasing the number of false positive results; this reduces the sensitivity of the test, thereby precluding the assay from accurately detecting small concentrations of target nucleotide in the sample.

C. Brakel et al., EPO Appln. 0330221, describes oligonucleotides with at least one biotin attached at each end thereof and an assay system for use therewith.

J. Tada et al., *Molec. and Cellular Probes* **6**, 489 (1992), describes an assay employing an amplification primer which is labelled with biotin at the 5' end thereof.

In view of the foregoing, it is a first object of the present invention to provide a sandwich assay with reduced non-specific background signal.

A second, object of the present invention to provide a kit suitable for expeditious performance of sandwich assays with reduced non-specific background signal.

A third object of the present invention is to provide probes and probe sets useful for carrying out sandwich assays with reduced non-specific background signal.

### Summary of the Invention

A method of detecting the presence of a target polynucleotide in a sample is disclosed. The method comprises the steps of:
(a) hybridizing the target polynucleotide with a detector probe, the detector probe comprising a first oligonucleotide which hybridizes to a first segment of the target polynucleotide and a detectable group attached to the 3' terminus of the first oligonucleotide;
(b) hybridizing the target polynucleotide with a capture probe, the capture probe comprising a second oligonucleotide which hybridizes to a second segment of the target polynucleotide and a capture group attached to the 5' terminus of the second oligonucleotide; and
(c) detecting the hybridization of the detector probe and the capture probe to the target polynucleotide.

A particular embodiment of the foregoing further comprises the step of attaching the capture probe to a solid support prior to the detecting step. For example, where the capture group is a first member of a specific binding pair, the solid support may have the second member of the specific binding pair bound thereto, and the attaching step may be carried out by binding the first and second members of the specific binding pair to one another. The detecting step can then be carried out by detecting the connecting of the detectable group to the solid support through the hybridization complex.

The foregoing and other objects and aspects of the present invention are described in detail in the drawings herein and the specification set forth below.

### Brief Description of the Drawings

**Figure 1** is a schematic illustration of a sandwich hybridization complex of a target polynucleotide, a detector probe, and a capture probe in accordance with the prior art; and
**Figure 2** is a schematic illustration of a sandwich hybridization complex of a target polynucleotide, a detector probe, and a capture probe in accordance with the present invention.

### Detailed Description of the Invention

Nucleotide sequences are presented herein by single strand only, in the 5' to 3' direction, from left to right.

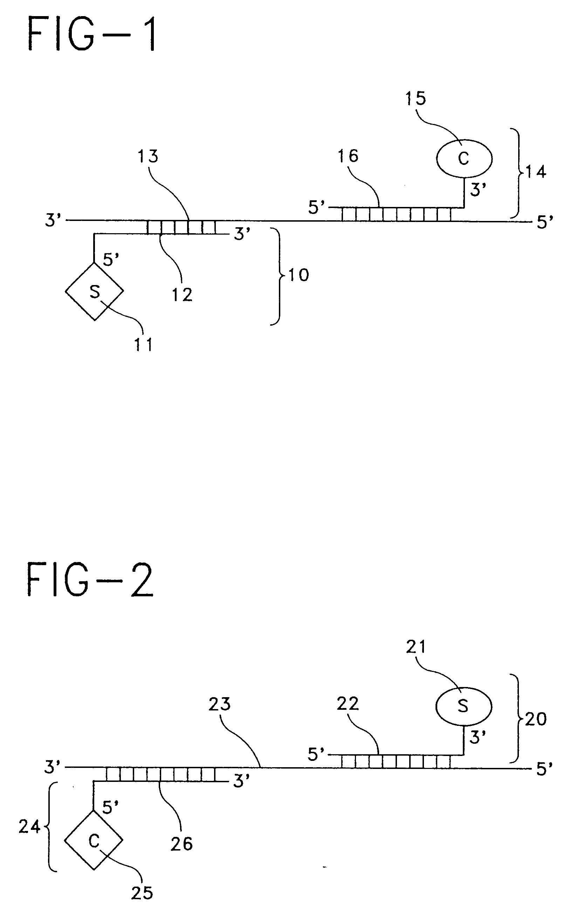

For comparative purposes, a hybridization complex formed in a prior art sandwich assay is schematically depicted in **Figure 1**. The bottom strand of **Figure 1** is the detector probe **10**, which includes a detectable group **11** on the 5' terminus of an oligonucleotide **12**. The center strand is the target polynucleotide **13**. The top strand is the capture probe **14**, which includes a capture group **15** on the 3' terminus of an oligonucleotide **16**. The short vertical lines connecting the detector probe 10 and the capture probe 14 to the target polynucleotide represent the hydrogen bonds formed during hybridization between complimentary base pairs.

A hybridization complex formed in a sandwich assay of the present invention is schematically depicted in **Figure 2**. This illustration is similar to Fig.1. Importantly, however, in Figure 2 the top strand is the detector probe **20**, and has a detectable group **21** on the 3' terminus of an oligonucleotide **22**. The center strand is the target polynucleotide **23**. The bottom strand of Fig. 2 is the capture probe **24**, which has the capture group **25** on the 5' terminus of an oligonucleotide **26**. The aspects of this method are discussed in detail below.

The target polynucleotide can be virtually any polynucleotide desired to be detected. For example, the target can comprise deoxyribonucleic acid (DNA), ribonucleic acid (RNA), and analogs and molecular complexes thereof. The target can comprise naturally occurring polynucleotides or polynucleotides that are produced by other means, such as recombinant techniques and polymerase chain reaction methods. The polynucleotide of interest can comprise virtually all of the polynucleic acids present in a sample or can comprise any majority or minority portion thereof. Also, the sample need not contain only polynucleic acids, but may and generally will contain other biomaterial. For example, this assay can be used to detect the presence of specific polynucleotides in whole or fractionated cells in culture, viri, bacteria, fungi, algae, yeasts, and other microorganisms. Commonly, these sources are found in samples taken from the blood, urine, feces, saliva, pus, semen, serum, or other tissue of an organism; thus the use of this assay to detect the presence of polynucleotides specific to one of these sources in a tissue sample can indicate the presence of the source itself in the sample. Bacteria such as *B*-hemolytic stretococci, Haemophilus *influenzae*, pneumococci, *Mycoplasma pneumoniae,* mycobacteria, salmonellae, shigellae, *Yersinia enterocolitica, Escherichia coli, Clostridium difficile,* Campylobacter, *Neisseria gonorrhoeae, Treponema* pallidun *, Chlamydia trachomatis, Clostridium perfringens* can be detected with the present invention. Exemplary viri suitable for this assay include influenza A, influenza B, parainfluenza, respiratory syncytial virus, adenoviri, rhinoviri, rotaviri, parvoviri, enteroviri, and Herpes simplex virus.

The target polynucleotide should be in single-stranded form for hybridization, but can be present in the hybridization solution in double-stranded form as long as the double strand is denatured to single strands prior to hybridization of the single strands with the detector probe. Any denaturing technique that is compatible with hybridization of the target and detector probe, the attachment of the target to the solid support, or the detection of the signalling moiety is suitable for use in the assay.

The target polynucleotide may be an amplicon produced by any suitable technique, such as strand displacement amplification or polymerase chain reaction. *See, e.g.,* G. Terrance Walker et al., *Nucleic Acids Res.* **20**, 1691-1696 (1992); K. Mullis et al., U.S. Patent No. 4,683,202. Thus, the target polynucleotide may be either natural or synthetic.

The target polynucleotide need only be sufficiently long to simultaneously bind two different oligonucleotide probes, with or without overlap of those probes. Typically the target nucleotide is at least 20 nucleotides in length. The target polynucleotide may be as long as genomic DNA, but typically the target polynucleotide is not more than 3,000 nucleotide bases in length. The first and second segments of the polynucleotide noted above refer to discreet regions of the polynucleotide to which a particular probe may hybridize; the first segment may be positioned in the polynucleotide either 3' or 5' to the second segment.

A detector probe, as noted above, comprises a first oligonucleotide which hybridizes to a first segment of the target polynucleotide and a detectable group attached to the 3' terminus of the first oligonucleotide. The length of the first oligonucleotide is not critical so long as it is capable of binding to a first segment of the target polynucleotide. The first oligonucleotide is typically 5, 6, 7, or 8 nucleotide bases in length up to 25, 30, and even 40 or more bases in length. The first oligonucleotide may be comprised of DNA or RNA, and may be synthetic or naturally occuring. *See generally* J. Goodchild, *Bioconjugate Chemistry* **1**, 165-187 (1990). Any suitable detectable group may be employed, examples including, but not limited to, of enzyme labels (*e.g.,* alkaline phosphatase, peroxidase such as horseradish peroxidase, acid phosphatase, β-D-galactosidase, glucose oxidase, luciferase), chemiluminescent labels (*e.g.*, luminols, lucigenin, acridinium compounds such as acridinium esters as described in L. Arnold et al., PCT Appln WO 89/02896), bioluminescent labels (*e.g.,* photoproteins such as aequorin and luciferase), fluorescent labels (*e.g.,* fluorescein), and electron dense labels (*e.g.,* ferritin, gold). Typically, the detectable group is a protein. The detectable group may be attached to the first oligonucleotide by any suitable technique. *See, e.g.,* S. Ghosh, *Bioconjugate Chemistry* **1**, 71-76 (1990).

Preferably, the detectable group used is alkaline phosphatase, and the preferred substrate is a combination of a dioxetane compound and a fluorescent compound such as fluorescein, with the reaction carried out under conditions permitting the activation of the dioxetane by the alkaline phosphatase and transfer of electronic energy from the dioxetane to the fluorescent compound, as described in U.S. Patent No. 4,959,182 to Schapp (the disclosure of all U.S. Patent references cited herein is to be incorporated herein by reference). Suitable dioxetane and fluorescent substrate systems are commercially available as LUMIPHOS™ 530 from Lumigen Inc., Detroit, Michigan, USA.

A capture probe, as also noted above, comprises a second oligonucleotide which hybridizes to a second segment of the target polynucleotide and a capture group attached to the 5' terminus of the second oligonucleotide. Again, the length of the second oligonucleotide is not critical so long as it is capable of binding to the second segment of the target polynucleotide. The second oligonucleotide, like the first, is typically 5, 6, 7, or 8 nucleotide bases in, length up to 25, 30, and even 40 or more bases in length. Like the first, the second oligonucleotide may be comprised of DNA or RNA, and may be synthetic or naturally occuring. Any suitable capture group may be employed, examples including, but not limited to, biotin and avidin, antigen and antibody, antibody and antibody binding protein (e.g., Protein A, protein G, Protein V). In general, the capture group is one member of a specific binding pair.

The solid support can be any means that permits the attached capture probe-target-detector probe hybridization complex to be separated from unbound sample, detector probe and capture probe. The solid support may be composed of any suitable material, including glass, polystyrene, polyethylene, dextran, nitrocellulose, nylon, and polypropylene. The solid support may take any suitable form or shape, examples including beads, test tubes, microwells, membranes, and the like.

It is preferred that the solid support include a means for binding the capture probe (i.e., have a group capable of binding, directly or indirectly, to the capture group of the capture probe attached thereto). Any suitable technique may be employed. In a preferred embodiment where biotin is used as the capture group on the oligonucleotide probe, a complex comprising a protein such as bovine serum albumin (BSA) and biotin is fixed to the solid support by the protein, streptavidin is bound to the biotin, and binding sites on the streptavidin remain free to bind the biotin capture group on the oligonucleotide probe.

Hybridization of oligogonucleotide probes to target polynucleotides involves the non-covalent bonding of a nucleotide strand to a complimentary nucleotide strand following the Watson-Crick base-pairing of adenine (A) and analogs thereof to thymine (T) and analogs thereof or uracil (U) and analogs thereof, and the pairing of guanine (G) and analogs thereof to cytosine (C) and analogs thereof. Hybridization may occur with 100 percent of the bases of one strand pairing with the proper complimentary base of the other strand; hybridization may also occur where only 95, 85, or 75 percent or less of the bases of one strand are complimentary to the bases of the other strand. Where two separate probes hybridize to a single target polynucleotide, as in the present invention, the two probes may hybridize thereto at segments which are either immediately adjacent or separated by an intervening segment. The probes themselves may, in some cases, partially overlap, which is of no consequence so long as each probe hybridizes to the target polynucleotide. Conditions which permit hybridization to occur are well known.

In general, hybridization may be carried out in an appropriate hybridization solution, typically an aqueous solution, in accordance with known techniques. Typically a hybridization solution will contain 50 percent formamide, a sodium chloride-sodium citrate mixture, and a small percentage of DNA (often calf thymus or salmon sperm). The target, detector probe, and capture probe are added, denatured if necessary, and allowed to hybridize and attach to the solid support. Excess reagents and sample are separated from the hybridized complex for detection. Those skilled in the art will understand that the steps of hybridizing the detector probe to the target, hybridizing the capture probe to the target, and attaching the capture probe to the solid support can be carried out in any order; i.e., either hybridization step can precede the other without affecting the accuracy of the assay, and the capture probe can be attached first to the solid support prior to or after it hybridizes with the target. In a preferred embodiment, the capture probe and the detector probe are added to the target sample simultaneously and permitted to hybridize; the solution is added to the solid support for attachment of this complex through the attachment moiety of the capture probe.

The detecting step of the assay can be carried out by known techniques suitable for inducing the detectable group to produce a detectable signal in association with the hybridization steps. Generally this will comprise contacting the detectable group to a substrate upon which it can act, and then detecting the amount of substrate converted by the detectable group. For example, after hybridization in an aqueous hybridization solution and attachment of the capture probe to a solid support in contact with the solution, the solution may be separated from the solid support, and the attachment of the detectable group to the solid support through the hybridization complex measured (i.e., by bringing a substrate capable of generating a detectable signal in contact with the solid support).

Kits for detecting a specific target polynucleotide in accordance with the present invention may comprise a detector probe and/or a capture probe, and optionally includes a solid support as described above. The components of the kit are typically contained together in a common package, which may also include a sheet of instructions for carrying out the method or have such instructions printed thereon. The kit may also include a substrate for the detectable group of the detector probe capable of producing a detectable signal.

The present invention is explained in greater detail in the following non-limiting examples.

### EXAMPLE 1

### Preparation of 5'-Biotinylated Capture Oligodeoxynucleotide Probe

Oligodeoxynucleotide capture probes were synthesized as described below. First, a capture oligomer was prepared using a DNA synthesizer (Model 380B, Applied Biosystems, Foster City, CA), and Biotin ON™ reagent (Clonetech, Palo Alto, CA), which produced an oligomer with three biotin molecules (BBB) at the 5' terminus. The probe was purified by reverse phase High Pressure Liquid Chromatography (HPLC, Brownlee Lab Aquapore RP 300 Column - 220 x 4.6 mm, C8 column 7 particle, 300 Å pore size) with an ultraviolet (UV) monitor at 254 mm and a gradient of 14 to 44% Buffer B over one hour (Buffer B: 0.1M Triethylamine-Acetate pH 7 with 50% Acetonitrile; Buffer A: 0.1M Triethylamine-Acetate, pH 7) at a flow rate of 1 ml/minute.

### EXAMPLE 2

### Preparation of 3'-Alkaline Phosphatase Detector Oligodeoxynucleotide Probes

Oligodeoxynucleotide detector probes were synthesized using a DNA synthesizer (Model 380B, Applied Biosystems, Foster City, CA) and a 3'-amino-modifier column (Glenn Research, Sterling, VA). This method yielded oligodeoxynucleotides with 3' amine termini required for subsequent conjugation with a maleimide derivatized alkaline phosphatase detector enzyme.

Calf intestine alkaline phosphatase (AP, enzyme immunoassay grade, Boehringer Mannheim, Indianapolis, ID) was dialyzed overnight at 4°C against 50 mM potassium phosphate pH 7.5 and subsequently centrifuged to remove aggregates. The alkaline phosphatase (4 mL, 10 mg/mL) was combined with a solution of 40 µL of succinimidyl-4-(p-maleimidophenyl)butyrate (SMPB, obtained from Pierce, Rockford, MD, 50 mM) dissolved in N,N'-dimethylformamide (DMF, Aldrich, Milwaukee, WI) and allowed to react in the dark at room temperature for 30 minutes. The derivatized alkaline phosphatase was purified using a NAP-25 column (Pharmacia, Piscataway, NJ) previously equilibrated with 50 mM potassium phosphate pH 7.5 (degassed and purged with N₂). The absorbances of the NAP-25 column fractions were measured at 260 and 280 nm and the void volume peak was pooled. The concentration of derivatized alkaline phosphatase was determined by absorbance at 280 nm using an extinction coefficient of 0.75 mL/µmole cm⁻¹. Typically, about 170 nmoles of SMPB-derivatized alkaline phosphatase were obtained and stored on ice (less than 2 hours).

The 3' amino-oligodeoxynucleotide (98.4 µL of 508.2 µM, 50 nmoles) was diluted in 13.4 µL of 1M potassium phosphate (pH 7.2) and mixed with 27 µl of a solution of *n*-succinimidyl-3-(2-pyridyldithio)propionate (50 mM, SPDP, Pierce, Rockford, IL) diluted in DMF. This mixture was incubated in the dark for 1 hour at room temperature. A solution of dithiothreitol(DTT, 1M) in 50 mM potassium phosphate (pH 7.5) was added to the SPDP-oligodeoxynucleotide conjugate/DMF solution (final concentration of 0.1M DTT) and allowed to incubate for 15 minutes at room temperature. Reduction of the SPDP-derivitized oligodeoxynucleotide with DTT generates a free thiol group for reaction with SMPB-derivatized alkaline phosphatase. Excess DTT and 2-thiopyridone were separated from the derivatized oligodeoxynucleotide by elution over a NAP-25 column with 50 mM potassium phosphate (pH 7.5).

Within 10 minutes of purification, the reduced oligodeoxynucleotide was mixed with the SMPB-derivatized alkaline phosphatase. Rapid mixture of the reduced oligomer and the SMPB-derivatized alkaline phosphatase can prevent reoxidation of the thiolated oligomer. The resulting solution was incubated 2-4 hours at room temperature, then overnight at 4°C, and was then quenched by addition of 1/100th the original volume of 50 mM 2-mercaptoethanol in 50 mM potassium phosphate (pH 7.5). The crude conjugate was concentrated using a Centriprep 30™ centrifugal concentrator (Amicon, Danvers, MA) to approximately 2 ml. This material was further purified by HPLC using a DEAE-5PW column (7.5 mm x 7.5 cm), a gradient of 0 to 66% Buffer B (Buffer B: 20 mM Tris, 1M NaCl pH 7.5, Buffer A: 20 mM Tris pH 7.5) and a flow rate of 1 ml/minute. Absorbance was monitored at 254 mm. Fractions with A₂₆₀/A₂₈₀ equal to 1.0-1.1 correspond to the conjugate and were pooled. The protein concentration of the conjugated oligodeoxynucleotide was then determined (BCA Protein Assay Kit, Pierce, Rockford, IL).

The purified alkaline phosphatase detector oligodeoxynucleotide probe was diluted to 2 µM in 20 mM Tris, 1M NaCl, 0.05% sodium azide, 50 µg/ml sonicated salmon sperm DNA, pH 7.5, and stored thereafter at 4°C.

### EXAMPLE 3

### 5' Detector Probe Enzyme Activity

Activity of the alkaline phosphate (AP) detector oligodeoxynucleotide probes were determined as follows. The conjugate was diluted to 5 µg/ml in 50 mM Tris-HCl, 100 mM NaCl, 1 mM MgCl₂, 1 mg/ml BSA, pH 7.5. The substrate, 4-nitrophenylphosphate (pNPP, 5 mM), was dissolved in 1 M diethanolamine, 1 mM MgCl₂, pH 9.8. The conjugate (5 µl) was diluted into 2 ml of the substrate solution at 25 °C and the change in absorbance monitored at 405 nm using a Hewlet Packard 8452 spectrophotometer. The reaction rates were calculated from the linear region of the kinetic plots using the extinction coefficient of p-nitrophenol at 405 nm (18500 M⁻¹cm⁻¹). The specific activity of the alkaline phosphatase detector oligodeoxynucleotide probes were determined to be 850-1300 µmole/minute/mg.

### COMPARATIVE EXAMPLE A

### Preparation of 3'-Biotinylated Capture Oligodeoxynucleotides

3'-biotinylated capture oligodeoxynucleotides were synthesized using a DNA synthesizer (Model 380B, Applied Biosystems, Foster City, CA). 3'Biotin-ON CPG (controlled pore glass, Clonetech, Palo Alto, CA) was used to attach a biotin moiety at the 3' terminus of the oligodeoxynucleotide. Two additional biotins were then added to the 3' terminus using Biotin-ON phosphoramidite reagent (also from Clonetech). The oligodeoxynucleotides were then prepared using standard phosphoramidite reagents and cleaved from the solid phase to give crude 3'-biotinylated oligodeoxynucleotides. Purification was done by reverse phase High Pressure Liquid Chromatography (HPLC) (Brownlee Lab Aquapore RP 300 Column - 220 x 4.6 mm, C8 column 7 particle, 300 A pore size) with a UV monitor at 254 nm and a gradient of 14 to 44% Buffer B over one hour (Buffer B: 0.1M Triethylamine-Acetate pH 7 with 50% Acetonitrile; Buffer A: 0.1M Triethylamine-Acetate, pH 7) and a flow rate of 1 ml/minute.

### COMPARATIVE EXAMPLE B

### Preparation of 5'-Alkaline Phosphatase Detector Oligodeoxynucleotides

5'-alkaline phosphatase oligodeoxynucleotides were synthesized from 5'-amino-oligodeoxynucleotides prepared using a DNA synthesizer (Model 380B, Applied Biosystems, Foster City, CA). The reagent AminoLink II (Applied Biosystems, Foster City, CA) was used to place an amine group on the 5'-end of the oligodeoxynucleotide for subsequent conjugation with alkaline phosphatase as described above. The crude conjugate was dialyzed into 20 mM Tris pH 7.5 and concentrated using a Centriprep 30 (Amicon, Danvers, MA) to approximately 2 ml. The concentrated conjugate was then purified by HPLC using a DEAE-5PW column (7.5 mm x 7.5 cm) and a gradient of 0 to 66% Buffer B (Buffer B: 20 mM Tris, 1M NaCl pH 7.5, Buffer A: 20 mM Tris pH 7.5) and a flow rate of 1 ml/minute. Absorbance was monitored at 254 nm. The fractions were collected, the activity of the conjugate was determined, and the conjugate was stored as described above.

### EXAMPLE 4

### Preparation of Coated Microtiter Plates

Biotinylated bovine serum albumin (biotin*BSA) (Pierce, Rockford, IL) was diluted to 5 µg/ml in 0.3 M Glycine, pH 9.6 (BRL, Bethesda, MD., prepared using autoclaved water), pipetted into each well (200 µl/well) of microLITE1™ plates (Dynatech, Chantilly, VA), and incubated at 4°C overnight. The plates were washed twice (375 µl/wash) using FTA hemagglutination buffer, pH 7.2 (Becton Dickinson Microbiology Systems, Cockeysville, MD, prepared using autoclaved water). Streptavidin (50 µg/ml) in hemagglutination buffer was added to the biotin*BSA-coated microliter wells (100 µl/well). Plates were covered and incubated for 1 hour at 37°C. Unbound streptavidin was discarded by inversion and blocking buffer (300 µl/well) (hemagglutination buffer pH 7.2, 0.05% weight/volume (w/v) bovine serum albumin, Sigma Chemical Co., St. Louis, Mo.) was added. The plates were covered and incubated (30 min, 37°C), and the blocking buffer was discarded by inversion. Plates were washed twice with hemagglutination buffer (375 µl/well), then once using hemagglutination buffer with 2% w/v trehalose (375 µl/well) (Fluka, Ronkonkoma, N.Y.). Plates were dried for approximately 4 hours under vacuum below 0.5 Torr at 25°C, sealed in mylar pouches with desiccant, and stored overnight at room temperature prior to use. The plates were stored thereafter at between 2-8°C.

### EXAMPLE 5

### Microtiter Plate Assay Procedure

This assay uses synthetic target DNA designed to imitate the SDA product generated from *M. avium/intracellulare* genomic DNA. The target sequence within the genome was determined from the pMAV29 sequence described in D. Wirth et al., *Molecular and Cellular Probes* **4**, 87-105 (1990). A biotinylated (BBB) capture probe binds to the target DNA and the streptavidin on the microliter plate. A second detector probe conjugated with Alkaline Phosphatase (AP) binds to the target DNA. The alkaline phosphatase provides and opportunity for detection by means of colorimetry, fluorimetry, or chemiluminescence. This assay evaluates the significance of conjugating the probes with biotin or alkaline phosphatase at either the 5' or 3' ends of the oligomers.

The assay used the following probes and target for the detection of synthetic *M.avium/intracellulare* target DNA:
**(1)** BBB-GGGAACCGGTGACTC (**SEQ ID NO:1**) (where each B is biotin);
**(2)** CAAAAACCTTGCGGC-P (**SEQ ID NO:2**)(where P is alkaline phosphatase);
**(3)** P-GGGAACCGGTGACTC (**SEQ ID NO:3**)(where P is alkaline phosphatase);
**(4)** CAAAAACCTTGCGGC-BBB (**SEQ ID NO:4**)(where each B is biotin); and
**(5)** 
GACCCGACTTGTAAGAGCCGCAAGGTTTTTGGAGTCAC
CGGTTCCCACTCGCAGCCTGCGTCTTTT (SEQ ID NO:5)
SEQ ID NO:1 and SEQ ID NO:2 represent a pair of probes of the present invention; SEQ ID NO:3 and SEQ ID NO:4 represent a pair of probes of the prior art; SEQ ID NO:5 represents a synthetic target DNA.

Synthetic target DNA (SEQ ID NO:5) was diluted into 0.1 mg/ml sheared salmon sperm DNA (Sigma) in sterile siliconized tubes to produce solutions having the following concentrations: 1600, 400, 100, 25, 6.25, 0 attomoles of target DNA/50µl volume. Diluted synthetic target DNA was heated to 95°C for 3 minutes to denature the DNA. Tubes were cooled for 5 minutes at room temperature and then 50µl of the denatured DNA was added to each well. Each level of target DNA was assayed in triplicate. Immediately thereafter, 50µl/well of hybridization mix (1M sodium phosphate, 0.2% BSA, 40nM capture probe, 10nM detector probe) was added. The plate was covered and incubated for 45 minutes at 37°C. SEQ ID NO:1 and SEQ ID NO:2 probes were paired in one set of wells, and SEQ ID NO:3 and SEQ ID NO:4 probes were paired in a different set of wells. Three stringency washes (300µl/well) (10mM sodium phosphate pH 7, 0.1% w/v bovine serum albumin, 0.05% Nonidet P-40 (Sigma) were performed at room temperature. Each wash was allowed to remain in the microliter wells for 1 minute before removing. Lumiphos™530 (100µl/well, Lumigen Inc., Detroit Michigan) substrate was added, and the plates were covered and incubated for 30 minutes at 37°C. Luminescence was read on a microtiter plate luminometer (Labsystems, Research Triangle Park, NC) at 37°C, using a 2 second/well integration time. Results are given in **Table 1** below. The results indicated as much as a threefold reduction in background signal for wells assayed with a SEQ ID NO:1/SEQ ID NO:2 probe set (which utilizes a 5'-biotin moiety and a 3'-alkaline phosphatase moiety) compared to wells assayed with a SEQ ID NO:3/SEQ ID NO:4 probe set. Because the probe reagents have identical DNA sequences, the lower backgrounds can be attributed to differences in terminus conjugation.

**TABLE 1**

| **Average Relative Light Units (Signal)** | | | | | | | |
|---|---|---|---|---|---|---|---|
| Probe Set | Attomoles/Test | | | | | | CV% Range¹ |
| | 0 | 6.25 | 25 | 100 | 400 | 1600 | |
| SEQ ID NO:1 and SEQ ID NO:2 | 8.03 | 10.88 | 20.13 | 49.3 | 180.5 | 645.4 | 3.3-10.1% |
| SEQ ID NO:3 and SEQ ID NO:4 | 24.85 | 30.97 | 39.32 | 56.14 | 164.33 | 510.5 | 1.7-27.4% |

| | | | | | | | |
|---|---|---|---|---|---|---|---|
| ¹CV%, calculated for each standard (triplicates) | | | | | | | |

### EXAMPLE 6

### Assay Procedure with Varied Probe Sequences

Using the procedures described above in Examples 4 and 5, probes with a slightly altered sequence were used to detect the *M.avium/intracellulare* synthetic target DNA. Again, the probe sets compared the significance of 3' or 5' biotin or alkaline phosphatase conjugation. The probes used in this example were:
**(6)** BBB-AACCGGTGACTCCA (**SEQ ID NO:6**)(where B is biotin);
**(7)** AAAACCTTGCGGC-P (**SEQ ID NO:7**)(where P is alkaline phosphatase);
**(8)** AAAACCTTGCGGC-BBB (**SEQ ID NO:8**)(where B is biotin); and
**(9)** P-AACCGGTGACTCCA (**SEQ ID NO:9**)(where P is alkaline phosphatase).
SEQ ID NO:6 and SEQ ID NO:7 represent a probe set of the present invention; SEQ ID NO:8 and SEQ ID NO:9 represent a probe set of the prior art; SEQ ID NO:5 was again the target DNA. Data gathered in the assay are given in **Table 2** below.

**TABLE 2**

| **Average Relative Light Units (Signal)** | | | | | | | |
|---|---|---|---|---|---|---|---|
| Probe Set | Attomoles/Test | | | | | | CV% Range |
| | 0 | 6.25 | 25 | 100 | 400 | 1600 | |
| SEQ ID NO:6 and SEQ ID NO:7 | 9.02 | 15.96 | 37.83 | 125.97 | 477.7 | 1791 | 3.1-5.4% |
| SEQ ID NO:8 and SEQ ID NO:9 | 267.77 | 286.33 | 320.33 | 433.4 | 730.03 | 1936.33 | 0.35-5.9% |

The results again indicate that the probe set consisting of SEQ ID NO:8 and SEQ ID NO:9 (3'-biotin, 5'-alkaline phosphatase) show as much as a 30-fold higher backgrounds and as much as a 16-fold decrease in detection sensitivity compared to the probe set consisting of SEQ ID NO:6 and SEQ ID NO:7 (5' biotin, 3' alkaline phosphatase).

### EXAMPLE 8

### Assay for M. tuberculosis target DNA

As in the foregoing Examples, this experiment used synthetic target DNA. However, the target was designed to imitate the Strand Displacement Amplification (SDA; G.T. Walker, et al. (1992) PNAS 89, 392-396; G.T. Walker al. (1992) Nucleic Acids Res. 20, 1691-1696) product generated from Mycobacteria tuberculosis genomic DNA. The target region within the genome was derived from the IS6110 sequence obtained genomic DNA. The target region within the genome was derived from the IS6110 sequence described in Thierry et al., *Nucleic Acids Research,* **18:** 188 (1990). The assay methodology described above was utilized on the following target with the following probes to assess detection of synthetic *M.tb* target DNA:
**(10)** TATCCACCATACGGA-BBB (**SEQ ID NO:10**)(where each B is biotin);
**(11)** P-CGACCTGAAAGACGT (**SEQ ID NO:11**)(where P is alkaline phosphatase);
**(12)** BBB-CCTGAAAGACGTTAT (**SEQ ID NO:12**) (where B is biotin);
**(13)** CCACCATACGGATAG-P (**SEQ ID NO:13**)(where P is alkaline phosphatase); and
**(14)** 
GACACTGAGATCCCCTATCCGTATGGTGGATAAC
GTCTTTCAGGTCGAGTACGCCGTCTTTTT (SEQ ID NO:14).
SEQ ID NO:10 and SEQ ID NO:11 represent a probe set of the prior art; SEQ ID NO:12 AND SEQ ID NO:13 represent a probe set of the present invention; SEQ ID NO:14 represents a target DNA. The synthetic target DNA was diluted to 2000, 1000, 500, 250, 125, 62.5, 31.25, and 0 attomoles/50µl volume and each probe set was used in an assay of that target in essentially the same manner as described above. **Table 3** shows the data gathered in the assay.

**TABLE 3**

| **Average Relative Light Units (Signal)** | | | | | | | | | |
|---|---|---|---|---|---|---|---|---|---|
| Probe Set | Attomoles/Test | | | | | | | | CV% Range |
| | 0 | 31.25 | 62.5 | 125 | 250 | 500 | 1000 | 2000 | |
| SEQ ID NO:10 and SEQ ID NO:11 | 19.87 | 25.1 | 36.9 | 56.4 | 104 | 202 | 420 | 856 | 2.8-13.39 |
| SEQ ID NO:12 and SEQ ID NO:13 | 4.94 | 24.13 | 44.5 | 89.6 | 170.7 | 331.7 | 721.6 | 1498 | 1.78-9.98 |

Once again, the probe set with the 5'biotin and 3' alkaline phosphatase conjugation (SEQ ID NO:12 and SEQ ID NO:13) showed a significant decrease in background levels and an increase in the amount of specific signal generated.

The foregoing examples are illustrative of the present invention, and are not to be construed as limiting thereof. The invention is defined by the following claims, with equivalents of the claims to be included therein.

## Claims

1. A method for detecting the presence of a target polynucleotide in a sample, said method comprising the steps of:
(a) hybridizing said target nucleotide with a detector probe, said detector probe comprising a first oligonucleotide which hybridizes to a first segment of said target polynucleotide and a detectable group attached to the 3' terminus of said first oligonucleotide;
(b) hybridizing said target nucleotide with a capture probe, said capture probe comprising a second oligonucleotide which hybridizes to a second segment of said target polynucleotide and a capture group attached to the 5' terminus of said second oligonucleotide; and
(c) detecting the hybridization of said detector probe and said capture probe to said target polynucleotide.

2. A method according to Claim 1, wherein said detectable group is selected from the group consisting of enzyme labels, chemiluminescent labels, bioluminescent labels, fluorescent labels, and electron dense labels.

3. A method according to Claim 1, wherein said capture group is a member of a specific binding pair.

4. A method according to Claim 1, wherein said capture group is selected from the group consisting of biotin and avidin.

5. A method according to Claim 1, further comprising the step of attaching said capture probe to a solid support.

6. A kit useful for detecting the presence of a target polynucleotide in a sample, said kit comprising:
(a) a detector probe, said detector probe comprising a first oligonucleotide which hybridizes to a first segment of said target polynucleotide and a detectable group attached to the 3' terminus of said first oligonucleotide;
(b) a capture probe, said capture probe comprising a second oligonucleotide which hybridizes to a second segment of said target polynucleotide and a capture group attached to the 5' terminus of said second oligonucleotide; and
(c) solid support means for binding said capture group.

7. A probe set useful for detecting the presence of a target polynucleotide in a sample, said probe set comprising:
(a) a detector probe, said detector probe comprising a first oligonucleotide which hybridizes to a first segment of said target polynucleotide and a detectable group attached to the 3' terminus of said first oligonucleotide;
and wherein said detectable group is selected from the group consisting of enzyme labels, chemiluminescent labels, bioluminescent labels, fluorescent labels, and electron dense labels; and
(b) a capture probe, said capture probe comprising a second oligonucleotide which hybridizes to a second segment of said target polynucleotide and a capture group attached to the 5' terminus of said second oligonucleotide.

8. A probe set according to Claim 7, wherein said detectable group is selected from the group consisting of alkaline phosphatase, horseradish peroxidase, luciferase, and acridinium compounds.

9. A probe set according to Claim 7, wherein said capture group is a member of a specific binding pair.

10. A probe set according to Claim 7, wherein said capture group is selected from the group consisting of biotin and avidin.
